# EUROPEAN PATENT APPLICATION

(11) **EP 2 979 662 A1**
(43) Date of publication of application: **03.02.2016**
(21) Application number: 15179175.3
(22) Date of filing: 30.07.2015
(51) Int. Cl.: A61F 2/16

(54) **INTRAOCULAR LENS WITH ELECTRICITY GENERATOR AND ADDITIONAL FUNCTIONAL SYSTEMS**

(30) Priority: 01.08.2014 NL 2013291
(71) Applicant: AKKOLENS INTERNATIONAL B.V., 4836 BA Breda (NL)
(72) Inventor: SIMONOV, Aleksey Nikolaevich, 2497 DV Den Haag (NL); ROMBACH, Michiel Christiaan, 4836 BA Breda (NL)
(74) Representative: Patentwerk B.V.

(57) **Abstract**

Accommodating intraocular *lens combination* with two optical elements (1,2) which move versus each other with the optical elements with such a shape that the combination provides variable focusing power at a different positions of at least one of the optical elements relative to the other optical element with the *lens combination* also comprising a electricity *generator (5,6)* and a *functional system (8),* which can be a, micro, mano meter system, or, a micro glucose measurement system, or, a micro intraocular driver system for amplification of accommodation movements. The *lens combination* comprises a wireless connection component to send or receive information from or to, for example, a smart-phone.

## Description

The invention disclosed in the present document concerns an intraocular combination, henceforth: intraocular *lens combination*, which combination includes (1) an intraocular accommodating lens, henceforth: *optical system,* as in, for example, US 20080046076, (2) at least one intraocular electricity generator, *generator,* which is a fully novel invention disclosed herein, and (3) at least one intraocular functional system, *functional system* (for example, a system which monitors intraocular glucose levels or, alternatively, monitors intraocular pressure).

Intraocular lenses, " IOLs", are surgically implanted to replace the natural lens of the eye. Standard monofocal IOLs provide the eye with only one focus with the eye generally being focused at distance and the wearer needs spectacles for close-up vision. Multifocal IOLs, "MIOLs", provide the eye with two or three foci, for example, a focus at far, middle and close-up, albeit at the cost of a reduced visual acuity, but the wearer is, likely, less spectacle dependent. By far the best solution for sharp vision at all distances are the novel, accommodating, IOLs, "AIOLs", as disclosed in, for example, US 20080046076, which provide the eye with a, continuous, change of focus, a change comparable to what the natural lens of the eye provides.

In the invention disclosed in the present document not only provides said continuous focus but also provides electrical energy, electrical current, to drive at least one *functional system.*

For example, March et al. (Diabetes Technol Ther. 2000 Spring; 2(1):27-30) successfully polymerized a fluorescent complex within a hydrogel intraocular lens that responds well to glucose concentration. The sensing module was proposed to also measure interleukin-2, interleukin-6, inter leukin- 10, interleukin-12, interferon-y, tumor growth factor- 2, tumor necrosis factor- , macrophage migration inhibitory factor, 8-Hydroxy-2'-deoxyguanosine, aB-crystallin, a-enolase, glial fibrillary acidic protein, pentosidine, N-carboxymethyl-lysine, monocyte chemoattractant protein- 1, interleukin-8, interphotoreceptor retinoid-binding protein, survivin, VEGF, amyloid-β, intercellular adhesion molecule- 1 (ICAM1), TNF-a, TGF-beta3, or transforming growth factor-beta2.

Clearly, with current micro technologies, including but not restricted to MEMS, MES, MEOS and micro fluidic technologies a whole battery of tests can be compressed into a minute micro component the size of, say, a grain of sugar, which is the size which can be integrated with an intraocular lens.

In the present document we focus on two main applications, the applications being measurement of glucose levels for diabetes management and measurement of intraocular pressure for management of macula degeneration, AMD, both applications of crucial importance for, elderly, cataract, and for, generally, somewhat younger, presbyope patients.

US 20040152963, which document is included in this present document, in full, by reference, discloses an apparatus for measuring ocular and/or blood glucose levels by irradiating means, irradiating light onto the eye from outside the cornea of the eye to excite an ocular glucose sensor in contact with an ocular fluid which ocular glucose concentration can be converted into a blood glucose concentration, the glucose sensor can be calibrated to reflect blood glucose levels based on the measured ocular glucose levels. So, the technology is, in the above example, albeit, in this example, in part, extra ocular embodiment, currently available. The present invention positions all required components inside the eye, as a fully integrated intraocular assembly, as in said, herein disclosed, *lens combination.*

Diabetes management via measurement of ocular glucose levels, by an intraocular micro glucose sensor, and management of macular degeneration via measurement of intraocular pressure, by an intraocular micro manometer are the primary application of the present invention, with all required components positioned inside the eye in the said *lens combination.*

The main hurdle to be taken at present is the provision of intraocular energy, meaning: electricity in the eye, to drive the proposed intraocular micro systems, MEMS, MES, MEOS or micro fluidic. To solve the energy issue intraocular fuel cells have been proposed (as in, for example, WO 2013040079) to provide such energy as are solar cells (as in, for example, WO 2013040079).and batteries, capacitors, loaded by external induction (as in, for example, WO 2006050171). The present invention solves this issue of energy by a dynamo, driven by the ciliary muscle of the eye during the process of accommodation of the eye, in combination with, for example, a micro capacitor or micro battery to store electrical energy for future use.

The *lens combination* is implanted in the human eye and provides accommodation of the eye by movement of the *optical system* by a natural component of the eye, for example, the ciliary muscle of the eye. The *optical system* comprises at least two optical elements of which at least one optical element is adapted to move versus the other optical element with the optical elements having such a shape that the combination of optical elements provides a, continuous, variable lens providing different focusing powers at different positions of at least one of the optical elements relative to the other optical element. Said movement by a natural component of the eye also drives the *generator* which provides electrical current, with the option of storing electrical energy in a micro capacitor or micro battery, while at least one *functional system* is driven by said current to provide an additional function.

The *optical system* can include a variable lens with at least two optical elements of which at least one optical element moves along the optical axis, as in, for example, US2012310342, which design comprises two, basically, spherical lenses in a telescope configuration.

Alternatively, the *optical system* can include a variable lens with at least two optical elements of which at least one optical element shifts in a direction perpendicular to the optical axis with each of the optical elements having at least one free-form optical surface with such a shape that the combination of at least two free-form surfaces provides different focusing powers, provides a variable lens, at a different position of at least one of the optical elements relative to the other element. Such intraocular variable lenses are well known from, for example, WO2005084587, NL1028496, US 20080046076, and WO2006118452 and other documents related thereto, and such optical design will be put forward henceforth to illustrate various embodiments of the novel *lens combinations* as set forth in the present document.

The intraocular *generator* provides electrical current and is driven by a natural component of the eye. The *generator* can comprise, for example, at least one, classical dynamo, for example, comprising a, thin and foldable, spool, coil, connected to the first optical element and at least one, thin and foldable, magnet connected to the second optical element to generate current, with the spool and magnet preferably positioned around the rim of the optical elements to maximize efficiency. The *generator* can also include a micro-battery, or micro-condensator, to store excess electrical energy for future use. Note that all components of any intraocular lens, which has a diameter of ∼6mm, must be thin and foldable as to allow injection into the eye of the wearer during surgery though a small incision of ∼2.5mm.

Alternatively, such *generator* can comprises at least one piezo-electrical dynamo to generate current, or, alternatively, such *generator* can comprise any other method by which electrical current can be generated by said movement of the optical elements.

The current can be used to drive at least one intraocular *functional system,* for example, an intraocular MEMS (micro-electrical-mechanical-system), or, alternatively, MES (micro-electronic-system), or, alternatively, a MEOS (micro-electronic-optical-system), or, alternatively, a MFS, micro fluidic system, or, multiple of such *functional systems* in any combination.

Such a function could be, for example, a micro intraocular mano-meter system to measure intraocular pressure, IOP, for management of macula degeneration. Such mano-meter can be fitted with electronics to connect the meter, wirelessly, with an external, meaning outside the eye, electronic read-out apparatus by wireless connection for continuous monitoring of intraocular pressure, for example, a wireless connection to a smart-phone.

Alternatively, the current can be used to drive at least one *functional system* to measure blood sugar levels, glucose levels, in the eye of the wearer of said intraocular accommodating *lens combination*. The system can be any standard glucose measurement component as long as it is small enough to fit the dimensions of the eye, or, alternatively, a combination of a light source and a micro spectrometer or other sensor, for example at least one laser or photodiode of which the beam shines through at least one section of at least one optical element, for example the light to transverse a path perpendicular to the optical axis within an optical element, or to transverse the liquid of the eye to reach a spectrometer sensor. For example, the micro laser or light source, for example, a micro photodiode, can be fitted opposite, for example, a fluorescence or even a Raman spectrometer making the glucose measurement from liquid inside the lens material. In such embodiment the glucose meter can be fully integrated inside the *lens combination*, for example, inside a moulded *lens combination*. With such embodiment almost continuous real-time glucose monitoring is achievable. Note that changing glucose levels inside a number of intraocular lens materials, for example many hydrophilic acrylic materials, relate to glucose levels in the eye which, in turn, relate to glucose levels in the blood. So, calibration can predict blood glucose levels based on glucose levels in the eye based on glucose levels in the intraocular lens.

Alternatively, the current can be used to drive an intraocular mechanical driver system, for example, at least one a micro piezo element, for driving the optical function of the accommodating lens. For example, a linear actuator, for example a linear piezo actuator, can be fitted to the lens such that, minor, movement of the ciliary muscle, the muscle which drives accommodation, is amplified, leveraged, in a, major, movement of the optical elements to reach a desired degree of accommodation, so, the system is adapted to adapted to amplify accommodation movements of the natural driving system of the eye.

Alternatively, the current can be used to drive an intraocular drug-delivery system with the component connected to an external electronic apparatus by wireless connection, so drugs can be delivered over time at the precise dosages required, for example, insulin to adjust blood sugar levels to treat diabetes, with dosages based on said intraocular glucose measurements.

Also, alternatively, the current can be used to drive an intraocular range-finder component, for example, by at least sensors, on each optical element, which report the precise position of the optical elements versus each other when the wearer focuses at a distant plane. The distance of the object can then be calculated with traditional standard optical formulas. This application can be of use for, for example, the military, and likely by players of the royal sport of golf, although the rules of the Royal & Ancient Golf Club of St Andrews specifically forbid any electronic, laser based or otherwise, range finding.

Finally, the current can drive multiple such *functional systems,* and the *functional systems* can comprises at least one electronic component to provide a wireless connection to send digital data to at least one extraocular receiver, for example, a smart-phone, or, alternatively, receive data send by an extraocular sender, or, alternatively, perform both these said functions. Also, the *generator* and the *functional system* can be an integral part of the accommodating *lens combination*, for example, be fully embedded in the lens material during a moulding manufacturing process, or, alternatively, be separately assembled after the *optical systems* is manufactured.

The Figures illustrate several embodiments of the inventions disclosed in the present document integrated with an accommodating lens, with the *optical system* as set forth in WO2005084587, NL1028496, and WO2006118452.

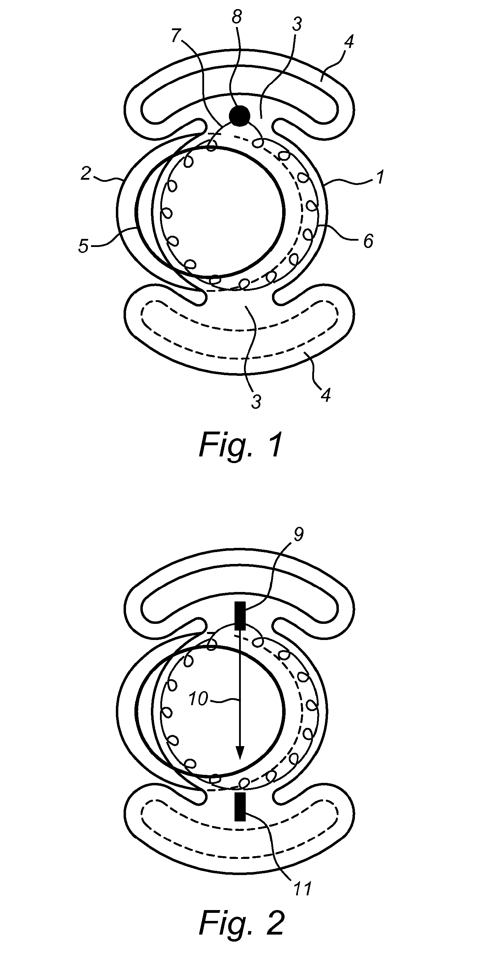

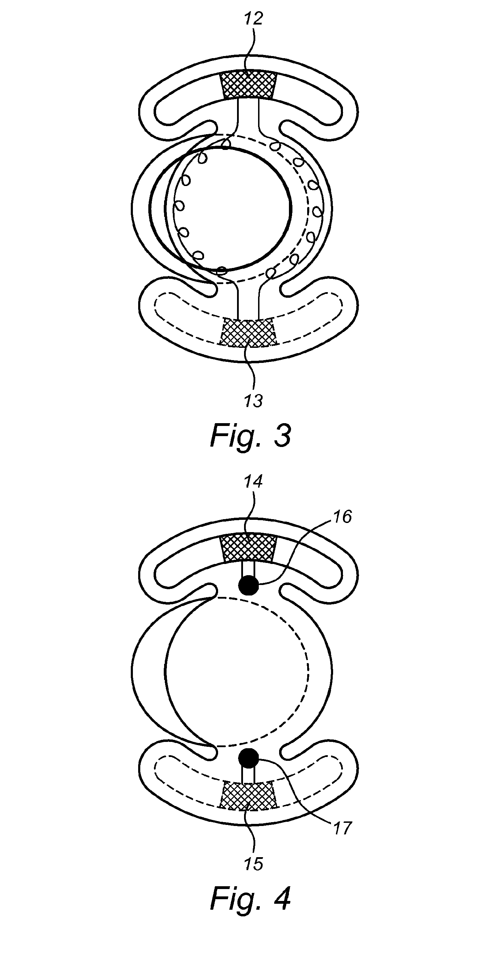

*Figure 1* The *lens combination* comprises two optical elements, 1, 2, and a connecting element, 3, which connects the optical elements (for optical function) to the haptics (components for mechanical function, for example, for connection of the *lens combination* to the ciliary muscle of the eye), 4. The *lens combination* further comprises, in this embodiment, for electricity generation, and generator, in this example a thin and foldable magnet, 5, and a, thin and foldable electrical coil, spool, 6, which components generate current when shifted versus each other. A micro battery or micro condensator can be added to the *generator* to store excess energy for future use. Connection wires, 7, connect the coil, spool, to the functional component, 8. *Figure 2* (see also Figure 1): illustrates an embodiment of a glucose measuring system, with a micro laser, 9, which generates at least one light beam, 10, which, in this example, shines at least one beam across and through the inside of the optical element to reach the, for example, receiving component, 11, for example, a fluorescence or Raman sensor. *Figure 3* (see also Figure 1): illustrates an embodiment embodiment in which the current drives two piezo actuators and accompanying electronics, 12, 13, in this embodiment fitted into the hinges of the accommodating lens, which piezo elements are designed to amplify, minor, movement of the natural driver of the lens in to amplified, major, movement of the optical elements. *Figure 4* (see also Figure 1) illustrates an embodiment in which current is generated by two piezo-dynamos, 14, 15, which are fitted in the hinge of the *lens combination* and which drive the *functional systems* 16 and 17.

So, the *lens combination* includes at least one intraocular *optical system* which includes at least two optical elements of which at least one optical element moves versus the other optical element with the optical elements having such a shape that the combination of the optical elements provides a variable, continuous, lens providing different focusing powers at a different positions of the optical elements relative to each other with the *lens combination* also comprising at least one electricity *generator* generating electrical current and at least one *functional system* providing an additional function, with the *generator* providing electrical current depending on the degree of compression and relaxation, depending on movement, of the optical elements, *optical system,* with the *generator,* for example, comprising at least one coil attached to the first optical element and at least one magnet attached to a second optical element with the combination of coil and magnet providing said electrical current, or, alternatively, with the *generator* comprising at least one piezo dynamo adapted to provide said electrical current, with the *generator* also including a micro battery or micro capacitor to store electrical current, and, with the *lens combination* also comprising at least one *functional system* providing at least one additional function, for example, at least one intraocular mano meter system providing the function of measuring intraocular pressure, or, alternatively, with the *functional system* comprising at least one intraocular glucose metering system providing the function of measuring intraocular glucose levels, or, alternatively, the *functional system* comprising at least one intraocular mechanical driver system providing the function of driving the *optical system,* by, for example, amplification of, minor, movements of at least one natural driving component of the eye into, major, movements of the *optical system,* or, alternatively, the *lens combination* comprising multiple *functional systems* providing multiple additional functions, with the *lens combination* also comprising at least one intraocular digital electronic component providing an, extra ocular, wireless connection to a receiving apparatus, or, alternatively, with the *lens combination* also comprising at least one intraocular digital electronic component providing an, extra ocular, wireless connection to a sending apparatus, which apparatus can be, for example, a smart-phone.

## Claims

1. *Lens combination* including at least one intraocular *optical system* which comprises at least two optical elements of which at least one optical element is adapted to move versus the other optical element with the optical elements having such a shape that the combination of the optical elements provides a variable, continuous, lens providing different focusing powers at a different positions of the optical elements relative to each other **characterized in that** the *lens combination* also comprises at least one electricity *generator* adapted to generate electrical current and at least one *functional system* adapted to provide an additional function.

2. *Lens combination* according to Claim 1 **characterized in that** the *lens combination* comprises at least one *generator* adapted to provide electrical current depending on the degree of compression and relaxation, on movement, of the *lens combination.*

3. *Lens combination* according to Claim 2 **characterized in that** the *generator* comprises at least one coil attached to the first optical element and at least one magnet attached to a second optical element with the combination of coil and magnet adapted to provide said electrical current.

4. *Lens combination* according to Claim 2 **characterized in that** the *generator* comprises at least one piezo dynamo adapted to provide said electrical current.

5. *Lens combination* according to any of the foregoing Claims **characterized in that** the *lens combination* also comprises at least one *functional system* adapted to provide at least one additional function.

6. *Lens combination* according to Claim 5 **characterized in that** the *functional system* comprises at least one intraocular mano meter system adapted to provide the function of measuring intraocular pressure.

7. *Lens combination* according to Claim 5 **characterized in that** the *functional system* comprises at least one intraocular glucose metering system adapted to provide the function of measuring intraocular glucose levels.

8. *Lens combination* according to Claim 5 **characterized in that** the *functional system* comprises at least one intraocular mechanical driver system adapted to provide the function of driving the *optical system.*

9. *Lens combination* according to Claim 8 **characterized in that** the driver system is adapted to amplify, minor, movements of at least one natural driving component of the eye into, major, movements of the *optical system.*

10. *Lens combination* according to Claim 5 **characterized in that** the *lens combination* comprises multiple *functional systems* adapted to provide multiple additional functions.

11. *Lens combination* according to any of foregoing Claims **characterized in that** the *lens combination* also comprises at least one intraocular digital electronic component adapted to provide an, extraocular, wireless connection.
